# EUROPÄISCHE PATENTANMELDUNG

(11) **EP 3 407 224 A1**
(43) Veröffentlichungstag der Anmeldung: **28.11.2018**
(21) Anmeldenummer: 17172455.2
(22) Anmeldetag: 23.05.2017
(51) Int. Cl.: G06F 19/00, G06Q 10/06, G06Q 50/22, A61B 6/03, A61B 6/00, A61B 5/055

(54) **FESTLEGUNGSVERFAHREN FÜR IMPLEMENTIERUNGEN VON UNTERSUCHUNGSAUFTRÄGEN**

(71) Anmelder: Siemens Healthcare GmbH, 91052 Erlangen (DE)
(72) Erfinder: Dominick, Lutz, 91330 Eggolsheim (DE); Ukis, Vladyslav, 90489 Nürnberg (DE)

(57) **Zusammenfassung**

Eine Recheneinrichtung (1) nimmt von einem Bediener (6) einen von einer bildgebenden medizintechnischen Einrichtung (2) (Modalität 2) auszuführenden Untersuchungsauftrag (U) entgegen. Der Untersuchungsauftrag (U) umfasst eine zu untersuchende Person individualisierende Daten (ident), die Person typisierende Daten (typ) und die Art der Untersuchung festlegende Daten (art). Die Recheneinrichtung (1) nimmt vom Bediener (6) eine zu realisierende Implementierung (I') des Untersuchungsauftrags (U) entgegen und ordnet sie dem Untersuchungsauftrag (U) zu. Die Recheneinrichtung (1) ermittelt anhand der die Person typisierenden Daten (typ) und der die Art der Untersuchung festlegenden Daten (art) unter Zugriff auf eine Datenbasis (7) mögliche Implementierungen (I) des Untersuchungsauftrags (U). Die Datenbasis (7) enthält aufgeschlüsselt nach die zu untersuchende Person typisierenden Daten (typ), Art der Untersuchung, Modalität (2) und Einstellungen (E) der Modalität (2) jeweils eine technisch-medizinische Bewertung (B1) der jeweiligen Untersuchung und eine betriebswirtschaftliche Bewertung (B2) der jeweiligen Untersuchung. Die Recheneinrichtung (1) gibt die ermittelten möglichen Implementierungen (I) dem Bediener (6) zusammen mit den beiden Bewertungen (B1, B2) zur Auswahl vor. Sie aktualisiert anhand der vom Bediener (6) entgegengenommenen zu realisierenden Implementierung (I') einen Ablaufplan (A) für die Modalität (2), so dass der Ablaufplan (A) zusätzlich zu bereits im Ablaufplan (A) enthaltenen Implementierungen (I') von anderen Untersuchungsaufträgen (U) auch den nunmehrigen Untersuchungsauftrag (U) und die zugeordnete zu realisierende Implementierung (I') enthält.

## Beschreibung

Die vorliegende Erfindung geht aus von einem Festlegungsverfahren für eine von einer bildgebenden medizintechnischen Einrichtung zu realisierende Implementierung eines Untersuchungsauftrags,
- wobei eine Recheneinrichtung von einem ersten Bediener den von der Einrichtung auszuführenden Untersuchungsauftrag entgegennimmt,
- wobei der Untersuchungsauftrag eine zu untersuchende Person individualisierende Daten, die zu untersuchende Person typisierende Daten und die Art der Untersuchung festlegende Daten umfasst,
- wobei die Recheneinrichtung vom ersten Bediener eine zu realisierende Implementierung des Untersuchungsauftrags entgegennimmt und die zu realisierende Implementierung des Untersuchungsauftrags dem Untersuchungsauftrag zuordnet.

Die vorliegende Erfindung geht weiterhin aus von einem Computerprogramm, das Maschinencode umfasst, der von einer Recheneinrichtung abarbeitbar ist, wobei die Abarbeitung des Maschinencodes durch die Recheneinrichtung bewirkt, dass die Recheneinrichtung ein derartiges Festlegungsverfahren ausführt.

Die vorliegende Erfindung geht weiterhin aus von einer Recheneinrichtung, wobei die Recheneinrichtung mit einem derartigen Computerprogramm programmiert ist, so dass sie im Betrieb ein derartiges Festlegungsverfahren ausführt.

Die Standardisierung im klinischen Arbeitsablauf (workflow) ist ein wichtiger Trend. Insbesondere muss beim Betrieb von Modalitäten (= bildgebende medizintechnische Anlagen wie beispielsweise CT-Scanner, MR-Scanner, C-Bogen-Anlagen) oftmals eine standardisierte Abfolge von Schritten eingehalten werden. Je größer eine Klinik bzw. ein Krankenhaus ist, desto starrer sind oftmals die einzuhaltenden Schritte vorgegeben. Die Schritte sind vorgegeben, um einerseits aus technisch-medizinischer Sicht gute Resultate wie beispielsweise eine hohe Bildqualität zu gewährleisten und andererseits aus betriebswirtschaftlicher Sicht einen effizienten Betrieb der Modalität zu gewährleisten. Ein weiterer Aspekt besteht insbesondere darin, eine Untersuchung zu vermeiden, die zu nicht verwertbaren Ergebnissen führt, weil die resultierenden Bilder qualitativ minderwertig sind. In diesem Fall wäre ein Rescan, d.h. eine Wiederholung der Untersuchung mit anderen Einstellungen der Modalität erforderlich. Ein Rescan ist sowohl aus medizinisch-technischer Sicht - beispielsweise aufgrund der zusätzlichen Strahlungsbelastung oder sonstigen physischen und psychischen Belastung des Patienten - als auch aus betriebswirtschaftlicher Sicht - es wird für das eigentlich gewünschte Ergebnis mehr Zeit benötigt - von Nachteil.

Die Aufgabe der vorliegenden Erfindung besteht darin, einen effizienten Betrieb bildgebender medizintechnischer Modalitäten zu gewährleisten.

Die Aufgabe wird durch ein Festlegungsverfahren mit den Merkmalen des Anspruchs 1 gelöst. Vorteilhafte Ausgestaltungen des Festlegungsverfahrens sind Gegenstand der abhängigen Ansprüche 2 bis 10.

Erfindungsgemäß wird ein Festlegungsverfahren der eingangs genannten Art dadurch ausgestaltet,
- dass die Recheneinrichtung anhand der die zu untersuchende Person typisierenden Daten und der die Art der Untersuchung festlegenden Daten des Untersuchungsauftrags unter Zugriff auf eine Datenbasis mögliche Implementierungen des Untersuchungsauftrags ermittelt,
- dass die Datenbasis aufgeschlüsselt nach die zu untersuchende Person typisierenden Daten, Art der Untersuchung, bildgebender medizintechnischer Einrichtung und Einstellungen der bildgebenden medizintechnischen Einrichtung jeweils eine technisch-medizinische Bewertung der jeweiligen Untersuchung und eine betriebswirtschaftliche Bewertung der jeweiligen Untersuchung enthält,
- dass die Recheneinrichtung die ermittelten möglichen Implementierungen des Untersuchungsauftrags dem ersten Bediener zusammen mit der jeweiligen technisch-medizinischen Bewertung und der betriebswirtschaftlichen Bewertung zur Auswahl vorgibt und
- dass die Recheneinrichtung anhand der vom ersten Bediener entgegengenommenen zu realisierenden Implementierung des Untersuchungsauftrags einen Ablaufplan für die bildgebende medizintechnische Einrichtung aktualisiert, so dass der Ablaufplan zusätzlich zu bereits im Ablaufplan enthaltenen Implementierungen von anderen Untersuchungsaufträgen auch den nunmehrigen Untersuchungsauftrag und die zugeordnete zu realisierende Implementierung des Untersuchungsauftrags enthält.

Durch diese Vorgehensweise wird zunächst gewährleistet, dass dem ersten Bediener von der Recheneinrichtung mögliche Implementierungen des Untersuchungsauftrags angeboten werden, die - sofern sie ausgeführt werden - zuverlässig zu medizinisch sinnvoll verwertbaren Ergebnissen führen. Weiterhin wird durch diese Vorgehensweise gewährleistet, dass der erste Bediener bei der Festlegung der Implementierung des Untersuchungsauftrags auch die erforderlichen betriebswirtschaftlichen Aspekte im Auge hat. Schließlich erfolgt eine automatische Einplanung der Implementierung des Untersuchungsauftrags in den Ablaufplan für die bildgebende medizintechnische Einrichtung, wobei in den Ablaufplan insbesondere auch die Einstellungen der bildgebenden medizintechnischen Einrichtung mit übernommen werden.

Vorzugsweise ist vorgesehen, dass die Recheneinrichtung anhand des Ablaufplans für die bildgebende medizintechnische Einrichtung die von dieser bildgebenden medizintechnischen Einrichtung jeweils als nächstes zu realisierende Implementierung ermittelt und die Einstellungen dieser bildgebenden medizintechnischen Einrichtung für die jeweils als nächstes zu realisierende Implementierung einem zweiten Bediener vorgibt. Dadurch können einerseits dem zweiten Bediener die Einstellungen der bildgebenden medizintechnischen Einrichtung vorgegeben werden, so dass der zweite Bediener die Einstellungen nur noch 1:1 übernehmen muss. Dennoch bleibt die menschlich-intellektuelle Kontrolle der bildgebenden medizintechnischen Einrichtung durch den zweiten Bediener aufrechterhalten.

Vorzugsweise ist weiterhin vorgesehen, dass die Recheneinrichtung mit einer Steuereinrichtung für die bildgebende medizintechnische Einrichtung datentechnisch verbunden ist und dass die Recheneinrichtung die Einstellungen der bildgebenden medizintechnischen Einrichtung für die von dieser bildgebenden medizintechnischen Einrichtung jeweils als nächstes zu realisierende Implementierung aufgrund einer Bestätigung durch den zweiten Bediener der Steuereinrichtung vorgibt. Dadurch gestaltet sich die 1:1-Übernahme (die stets oder zumindest im Regelfall folgen wird) besonders einfach.

Im Falle mehrerer bildgebender medizintechnischer Einrichtungen - insbesondere mehrerer gleichartiger bildgebender medizintechnischer Einrichtungen - ist vorzugsweise vorgesehen, dass die Recheneinrichtung die ermittelten möglichen Implementierungen des Untersuchungsauftrags dem ersten Bediener aufgeschlüsselt nach der jeweiligen bildgebenden medizintechnischen Einrichtung vorgibt. Dadurch kann die Recheneinrichtung vom ersten Bediener eine Vorgabe entgegennehmen, mit welcher bildgebenden medizintechnischen Einrichtung der Untersuchungsauftrag ausgeführt werden soll. Dies kann einerseits eine Auswahl der Art der bildgebenden medizintechnischen Einrichtung (beispielsweise CT-Scanner, MR-Scanner, C-Bogen-Anlage) und andererseits im Falle mehrerer gleichartiger bildgebender medizintechnischer Einrichtungen (beispielsweise mehrerer CT-Scanner) die Auswahl der spezifischen bildgebenden medizintechnischen Einrichtung, also beispielsweise welches CT-Scanners, sein. Selbstverständlich ist auch eine kombinierte Auswahl sowohl von Art der bildgebenden medizintechnischen Einrichtung als auch welcher von mehreren gleichartigen bildgebenden medizintechnischen Einrichtungen möglich.

Vorzugsweise ist weiterhin vorgesehen, dass die Recheneinrichtung anhand des Ablaufplans für die bildgebende medizintechnische Einrichtung, wie er der Recheneinrichtung vor der Entgegennahme des Untersuchungsauftrags vorliegt, für die möglichen Implementierungen des Untersuchungsauftrags jeweils einen möglichen Implementierungsbeginn ermittelt, zu dem die jeweilige mögliche Implementierung des Untersuchungsauftrags gestartet werden kann, und dass die Recheneinrichtung dem ersten Bediener zusammen mit den ermittelten möglichen Implementierungen des Untersuchungsauftrags auch den möglichen Implementierungsbeginn mit anzeigt. Diese Vorgehensweise ermöglicht dem ersten Bediener eine Abschätzung für die Wartezeit, die abgewartet werden muss, bis das Ergebnis der Untersuchung vorliegt. Diese Vorgehensweise ist insbesondere in Verbindung mit mehreren bildgebenden medizintechnischen Einrichtungen und der Möglichkeit zu deren Auswahl durch den ersten Bediener von Vorteil. Wenn - beispielsweise - bei der bildgebenden medizintechnischen Einrichtung A aufgrund deren Belegung lange gewartet werden muss, bis das Ergebnis der Untersuchung vorliegt, während bei der bildgebenden medizintechnischen Einrichtung B die Untersuchung sofort oder bald beginnen kann, kann der ersten Bediener in diesem Fall gezielt die bildgebende medizintechnische Einrichtung B auswählen.

Vorzugsweise ist vorgesehen, dass die Recheneinrichtung zur Entgegennahme der zu realisierenden Implementierung vom ersten Bediener eine Auswahl einer der möglichen Implementierungen entgegennimmt. Es ist möglich, dass der erste Bediener die jeweilige mögliche Implementierung nur so, wie sie ist, auswählen kann, also ohne Modifikation. Alternativ ist es möglich, dass der erste Bediener vor der (endgültigen) Auswahl noch Modifikationen vornehmen kann.

Vorzugsweise ist vorgesehen, dass die Recheneinrichtung den tatsächlichen Implementierungsbeginn des Untersuchungsauftrags durch die bildgebende medizintechnische Einrichtung und zusätzlich das tatsächliche Implementierungsende des Untersuchungsauftrags durch die bildgebende medizintechnische Einrichtung oder den tatsächlichen Implementierungsbeginn des nächsten Untersuchungsauftrags durch die bildgebende medizintechnische Einrichtung entgegennimmt. Dadurch ist es möglich, dass die Recheneinrichtung aus diesen Daten - also dem tatsächlichen Implementierungsbeginn des Untersuchungsauftrags durch die bildgebende medizintechnische Einrichtung einerseits und das tatsächliche Implementierungsende des Untersuchungsauftrags durch die bildgebende medizintechnische Einrichtung oder den tatsächlichen Implementierungsbeginn des nächsten Untersuchungsauftrags durch die bildgebende medizintechnische Einrichtung andererseits - die für die Ausführung der Implementierung des Untersuchungsauftrags durch die bildgebende medizintechnische Einrichtung erforderliche Zeitdauer abschätzt und anhand der Abschätzung der Zeitdauer die betriebswirtschaftliche Bewertung nachführt.

In analoger Weise ist vorzugsweise vorgesehen, dass die Recheneinrichtung die mittels der bildgebenden medizintechnischen Einrichtung erfassten Daten auswertet und/oder von einem dritten Bediener eine Bewertung entgegennimmt. In diesem Fall ist es möglich, dass die Recheneinrichtung die technisch-medizinische Bewertung anhand der Auswertung und/oder der vom dritten Bediener entgegengenommenen Bewertung nachführt. Der dritte Bediener kann mit dem ersten Bediener identisch sein.

Zur anfänglichen Ermittlung der betriebswirtschaftlichen Bewertung ist vorzugsweise vorgesehen,
- dass die Recheneinrichtung auf Datensätze bereits ausgeführter Untersuchungsaufträge zugreift,
- dass die bereits ausgeführten Untersuchungsaufträge jeweils die untersuchte Person typisierende Daten, die Art der Untersuchung, die bildgebende medizintechnische Einrichtung und die Einstellungen der bildgebenden medizintechnischen Einrichtungen sowie die Anfangszeiten der jeweiligen Untersuchungsaufträge umfassen,
- dass die Recheneinrichtung anhand der Anfangszeiten die Reihenfolge der bereits ausgeführten Untersuchungsaufträge und für den jeweiligen bereits ausgeführten Untersuchungsauftrag eine geschätzte Implementierungsdauer ermittelt und
- dass die Recheneinrichtung die geschätzten Implementierungsdauern der bereits ausgeführten Untersuchungsaufträge bei der Ermittlung der betriebswirtschaftlichen Bewertung berücksichtigt.

Hierbei ist es möglich, dass die im Rahmen der zuletzt genannten Vorgehensweise ausgewerteten Datensätze unabhängig von dem erfindungsgemäßen Festlegungsverfahren erstellt wurden.

Vorzugsweise ist vorgesehen,
- dass die Datenbasis aufgeschlüsselt nach die zu untersuchende Person typisierenden Daten, Art der Untersuchung und bildgebender medizintechnischer Einrichtung für die Einstellungen der bildgebenden medizintechnischen Einrichtung und/oder die technisch-medizinische Bewertung der jeweiligen Untersuchung und/oder die betriebswirtschaftliche Bewertung der jeweiligen Untersuchung jeweils einen üblichen Bereich enthält und
- dass die Recheneinrichtung dem ersten Bediener zusammen mit den ermittelten möglichen Implementierungen des Untersuchungsauftrags auch die Lage der Einstellungen der bildgebenden medizintechnischen Einrichtung und/oder der technisch-medizinischen Bewertung der jeweiligen Untersuchung und/oder der betriebswirtschaftlichen Bewertung der jeweiligen Untersuchung innerhalb des jeweils üblichen Bereichs mit anzeigt.

Dadurch ist es für den ersten Bediener auf einfache Weise möglich, zu erkennen, ob eine bestimmte mögliche Implementierung des Untersuchungsauftrags im Rahmen des Üblichen liegt. "Ausreißer" können dadurch ohne weiteres erkannt werden.

Die Aufgabe wird weiterhin durch ein Computerprogramm mit den Merkmalen von Anspruch 11 gelöst. Erfindungsgemäß bewirkt die Abarbeitung des Maschinencodes durch die Recheneinrichtung, dass die Recheneinrichtung ein erfindungsgemäßes Festlegungsverfahren ausführt.

Die Aufgabe wird weiterhin durch eine Recheneinrichtung mit den Merkmalen von Anspruch 12 gelöst. Erfindungsgemäß ist die Recheneinrichtung mit einem erfindungsgemäßen Computerprogramm programmiert, so dass sie im Betrieb ein erfindungsgemäßes Festlegungsverfahren ausführt.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Implementierungsbeispiele, die in Verbindung mit den Zeichnungen näher erläutert werden. Hierbei zeigen in schematischer Darstellung:
- FIG 1: einen Überblick über ein Gesamtsystem,
- FIG 2: ein Ablaufdiagramm,
- FIG 3: einen Untersuchungsauftrag,
- FIG 4: eine Datenbasis und
- FIG 5 bis 10: Ablaufdiagramme.

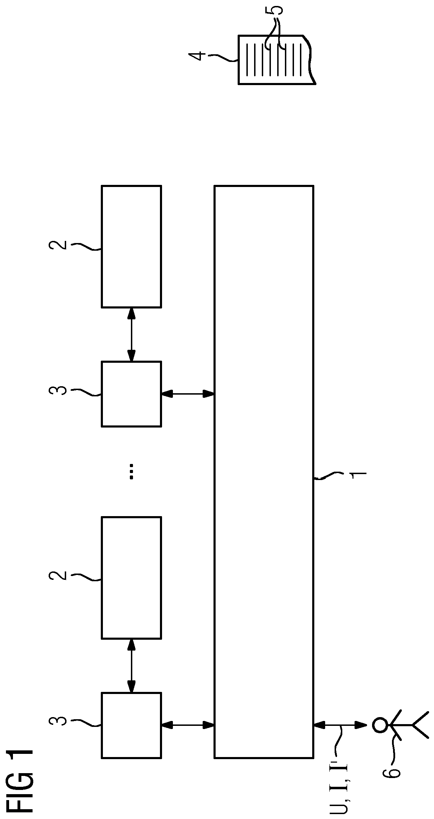

Gemäß FIG 1 ist eine Recheneinrichtung 1 im Umfeld eines Krankenhauses angeordnet. Innerhalb des Krankenhauses gibt es mindestens eine bildgebende medizintechnische Einrichtung 2, beispielsweise einen CT-Scanner, einen MR-Scanner, eine C-Bogen-Röntgenanlage oder eine ähnliche Einrichtung. Derartige Einrichtungen werden in Fachkreisen üblicherweise als Modalität bezeichnet. Es können auch mehrere Modalitäten 2 vorhanden sein. Es ist sogar möglich, dass mehrere gleichartige Modalitäten vorhanden sind, beispielsweise mehrere MR-Scanner. Die Modalität 2 wird von einer Steuereinrichtung 3 gesteuert.

Die Recheneinrichtung 1 ist mit einem Computerprogramm 4 programmiert. Das Computerprogramm 4 umfasst Maschinencode 5, der von der Recheneinrichtung 1 abarbeitbar ist. Die Abarbeitung des Maschinencodes 5 durch die Recheneinrichtung 1 bewirkt, dass die Recheneinrichtung 1 im Betrieb ein Festlegungsverfahren für eine zu realisierende Implementierung I' eines Untersuchungsauftrags U ausführt, wobei die zu realisierende Implementierung I' des Untersuchungsauftrags U von der Modalität 2 bewirkt werden soll.

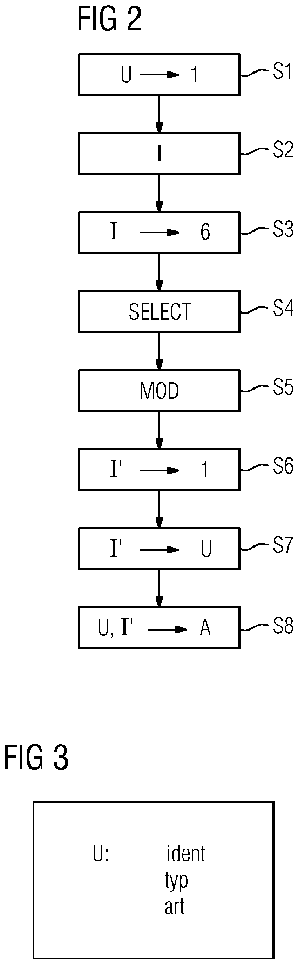

Im Rahmen des Festlegungsverfahrens nimmt die Recheneinrichtung 1 zunächst - siehe in FIG 2 einen Schritt S1 - von einem ersten Bediener 6 - beispielsweise einem Arzt - einen Untersuchungsauftrag U entgegen. Der Untersuchungsauftrag U umfasst gemäß FIG 3 Daten ident, typ und art. Die Daten ident sind Daten, welche eine zu untersuchende Person individualisieren. Sie können beispielsweise den Namen (gegebenenfalls aufgeschlüsselt nach Vorname und Familienname), eine Adresse, ein Geburtsdatum, eine nur einmal vergebene Nummer und dergleichen mehr umfassen. Die Daten typ sind Daten, welche die zu untersuchende Person typisieren. Die Daten typ können beispielsweise das Alter, das Geschlecht, das Gewicht, die Größe der zu untersuchenden Person und dergleichen mehr umfassen. Die Daten art legen die Art der Untersuchung fest. Die Daten art können beispielsweise eine Körperregion der zu untersuchenden Person und ein Diagnoseziel festlegen, beispielsweise ob die zu untersuchende Person auf einen Knochenbruch, eine Blutung oder ein Krebsgeschwür hin untersucht werden soll. Oftmals wird mittels der Daten art auch die bevorzugte Modalität 2 für die Untersuchung, unter Umständen sogar bei mehreren möglichen gleichartigen Modalitäten 2 eine ganz bestimmte dieser Modalitäten 2 festgelegt oder zumindest als bevorzugt markiert.

Zwischen den Daten ident und typ können gewisse Überschneidungen auftreten. So kann beispielsweise das Geschlecht sowohl zur Identifizierung bzw. Individualisierung der zu untersuchenden Person als auch zur Typisierung dieser Person verwendet werden. Gleiches gilt für das Geburtsdatum, welches in Verbindung mit dem aktuellen Datum das Alter bzw. die Altersklasse (= ungefähres Alter) bestimmt.

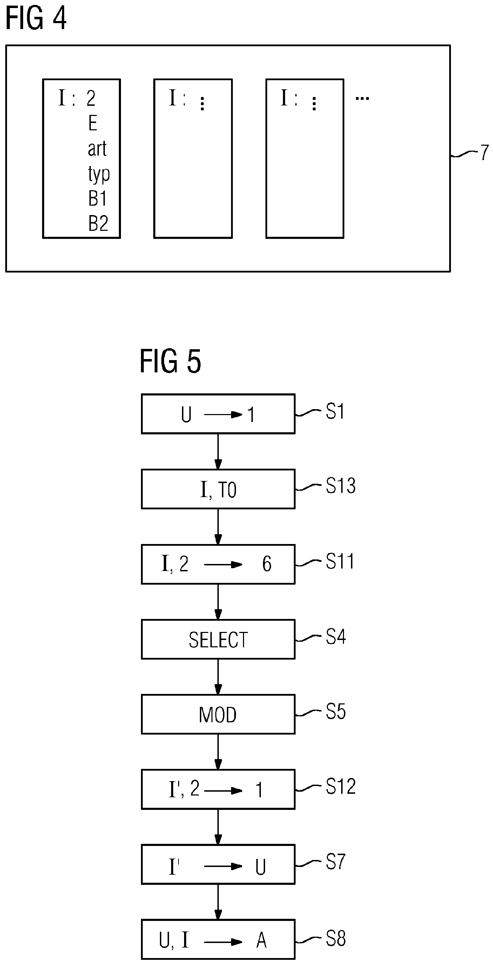

Anhand der Daten typ und art ermittelt die Recheneinrichtung 1 sodann in einem Schritt S2 - im Falle der Spezifikation der Modalität 2 zumindest und bevorzugt für diese Modalität 2 - mögliche Implementierungen I des Untersuchungsauftrags U. Die möglichen Implementierungen I umfassen gemäß FIG 4 jeweils die Modalität 2, mit welcher der Untersuchungsauftrag U ausgeführt werden soll, und die Einstellungen E der entsprechenden Modalität 2. Die Einstellungen E können statische und dynamische Daten umfassen, beispielsweise Sequenzprotokolle, zu verwendende Spannungen und Ströme und dergleichen mehr. Die Recheneinrichtung 1 ermittelt die möglichen Implementierungen I gemäß FIG 1 unter Zugriff auf eine Datenbasis 7. Die Datenbasis 7 enthält gemäß FIG 4 für jede in der Datenbasis 7 gespeicherte Implementierung I die Modalität 2 und die Einstellungen E der Modalität 2 sowie die Art der Untersuchung und die die zu untersuchende Person typisierenden Daten typ. Ferner enthält sie für jede Implementierung I eine technisch-medizinische Bewertung B1 und eine betriebswirtschaftliche Bewertung B2. Die technisch-medizinische Bewertung B1 gibt Aufschluss über die Qualität und die Ergebnisse, die erreicht werden, wenn die Modalität 2 mit den Einstellungen E betrieben wird, um eine Person mit den durch die Daten typ charakterisierten Eigenschaften auf die für die Implementierung I spezifizierte Art der Untersuchung zu untersuchen. Die betriebswirtschaftliche Bewertung B2 gibt Aufschluss über die Wirtschaftlichkeit des Betriebs der Modalität 2. Die betriebswirtschaftliche Bewertung B2 kann insbesondere anhand der Zeitdauer ermittelt werden, welche für die Ausführung der Implementierung I und damit für die Untersuchung erforderlich ist.

Die Datenbasis 7 enthält die Implementierungen I aufgeschlüsselt nach den genannten Parametern. Es ist also für bestimmte Daten typ, art nicht nur die Modalität 2 spezifiziert, sondern auch die Einstellungen E und die Bewertungen B1, B2. Vorzugsweise sind die Implementierungen I in der Datenbank 7 auch indexiert, so dass sie schnell und einfach aufgefunden werden können. Die Indexierung ist hierbei zumindest für die Daten typ, art gegeben. Sie kann zusätzlich auch für einige oder alle der anderen Parameter (Modalität 2, Einstellungen E und Bewertungen B1, B2) gegeben sein. Indexierung ist für Fachleute auf dem Gebiet von Datenbanken ein feststehender Begriff.

In einem Schritt S3 gibt die Recheneinrichtung 1 die ermittelten möglichen Implementierungen I des Untersuchungsauftrags U dem ersten Bediener 6 zur Auswahl vor. Die Vorgabe erfolgt zusammen mit der jeweiligen technisch-medizinischen Bewertung B1 und der betriebswirtschaftlichen Bewertung B2.

Es ist möglich, dass die Recheneinrichtung 1 vom ersten Bediener 6 sodann in einem Schritt S4 eine Vorselektion einer der möglichen Implementierungen I entgegennimmt und in einem Schritt S5 Modifikationen der vorselektierten Implementierung I entgegennimmt. Die Schritte S4 und S5 sind jedoch nur optional. Unabhängig davon, ob die Schritte S4 und S5 vorhanden sind, nimmt die Recheneinrichtung 1 jedoch in einem Schritt S6 vom ersten Bediener 6 eine zu realisierende Implementierung I' des Untersuchungsauftrags U entgegen. Die Entgegennahme kann insbesondere durch (endgültige) Auswahl einer der zuvor ermittelten (und gegebenenfalls modifizierten) möglichen Implementierungen I durch den ersten Bediener 6 erfolgen.

In einem Schritt S7 ordnet die Recheneinrichtung 1 die zu realisierende Implementierung I' des Untersuchungsauftrags U dem Untersuchungsauftrag U zu. Weiterhin aktualisiert die Recheneinrichtung 1 in einem Schritt S8 anhand der Implementierung I' einen Ablaufplan A für die Modalität 2. Der Ablaufplan A gibt an, in welcher Reihenfolge die einzelnen Untersuchungsaufträge U bzw. die zugehörigen Implementierungen I' ausgeführt werden soll. Die Recheneinrichtung 1 fügt dem Ablaufplan A den neu hinzugekommenen Untersuchungsauftrag U bzw. die zugehörige Implementierung I' also hinzu. Der aktualisierte Ablaufplan A enthält somit nunmehr zusätzlich zu bereits im Ablaufplan A enthaltenen Implementierungen I' von anderen Untersuchungsaufträgen U auch den nunmehrigen Untersuchungsauftrag U und die zugeordnete zu realisierende Implementierung I'.

In vielen Fällen ist nicht nur eine einzelne Modalität 2 vorhanden, sondern sind mehrere Modalitäten 2 vorhanden. In diesem Fall wird die Vorgehensweise von FIG 2 so modifiziert, wie dies nachfolgend in Verbindung mit FIG 5 näher erläutert wird.

Gemäß FIG 5 ist zum einen der Schritt S3 durch einen Schritt S11 ersetzt. Der Schritt S11 korrespondiert im Wesentlichen mit dem Schritt S3. Zusätzlich zur Vorgabe der ermittelten möglichen Implementierungen I gibt die Recheneinrichtung 1 im Schritt S11 jedoch auch eine Information über die jeweilige Modalität 2 aus, für welche die jeweilige mögliche Implementierung I gültig ist. Die möglichen Implementierungen I werden dem ersten Bediener 6 also aufgeschlüsselt nach der jeweiligen Modalität 2 vorgegeben. Weiterhin ist in diesem Fall der Schritt S6 durch einen Schritt S12 ersetzt. Im Schritt S12 nimmt die Recheneinrichtung 1 vom ersten Bediener 6 zusätzlich zu den Vorgaben des Schrittes S6 eine Vorgabe entgegen, mit welcher Modalität 2 der Untersuchungsauftrag U ausgeführt werden soll.

Vorzugsweise ermittelt die Recheneinrichtung 1 weiterhin entsprechend der Darstellung in FIG 5 zusammen mit den möglichen Implementierungen I in einem Schritt S13 (= Ersatz für den Schritt S2 von FIG 2) anhand des Ablaufplans für die jeweilige Modalität 2 (so wie der Ablaufplan A der Recheneinrichtung 1 vor der Entgegennahme des Untersuchungsauftrags U vorliegt) für die Implementierungen I auch jeweils einen möglichen Implementierungsbeginn T0. Der jeweilige Implementierungsbeginn T0 gibt an, zu welchem Zeitpunkt die jeweilige Implementierung I des Untersuchungsauftrags U an der jeweiligen Modalität 2 gestartet werden kann. In diesem Fall zeigt die Recheneinrichtung 1 dem ersten Bediener 6 zusammen mit den ermittelten möglichen Implementierungen I des Untersuchungsauftrags U auch den jeweils möglichen Implementierungsbeginn T0 mit an.

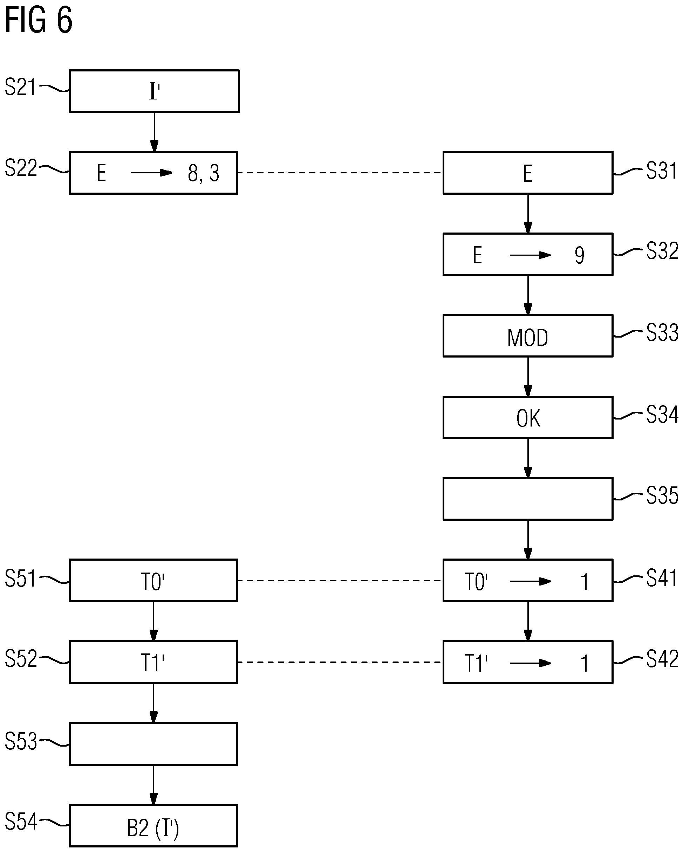

Mittels der Recheneinrichtung 1 erfolgt nicht nur die Ermittlung (unter anderem) des Ablaufplans A für die jeweilige Modalität 2. Anhand des Ablaufplans A für die (gegebenenfalls jeweilige) Modalität 2 ist die Recheneinrichtung 1 auch in der Lage, entsprechend der Darstellung in FIG 6 in einem Schritt S21 für die jeweilige Modalität 2 die jeweils als nächstes zu realisierende Implementierung I' zu ermitteln. Dadurch ist die Recheneinrichtung 1 auch in der Lage, die zugehörigen Einstellungen E der jeweiligen Modalität 2 für die als nächstes zu realisierende Implementierung I' in einem Schritt S22 einem zweiten Bediener 8 vorzugeben. Der zweite Bediener 8 kann beispielsweise ein medizinisch-technischer Assistent sein, der die entsprechende Modalität 2 über deren Steuereinrichtung 3 bedient und steuert.

Es ist möglich, dass im Schritt S22 eine direkte Vorgabe an den zweiten Bediener 8 erfolgt. Oftmals ist die Recheneinrichtung 1 jedoch mit der Steuereinrichtung 3 für die Modalität 2 datentechnisch verbunden. In diesem Fall kann die Recheneinrichtung 1 die Einstellungen E an die Steuereinrichtung 3 übermitteln. Die Steuereinrichtung 3 nimmt in diesem Fall die Einstellungen E in einem Schritt S31 entgegen und gibt sie in einem Schritt S32 über ein Bedienterminal 9 an den zweiten Bediener 8 aus. Der zweite Bediener 8 ist für die tatsächlichen Einstellungen E der Modalität 2 verantwortlich. Er ist daher in der Lage, die Einstellungen E in einem Schritt S33 zu modifizieren und in einem Schritt S34 zu bestätigen. Erst aufgrund der Bestätigung übernimmt die Steuereinrichtung 3 die jeweiligen Einstellungen E als endgültige Einstellungen E. In aller Regel ist der zweite Bediener 8 jedoch zwar in der Lage, im Schritt S33 Modifikationen vorzunehmen, nimmt jedoch rein faktisch keine Modifikationen vor. In einem Schritt S35 steuert die Steuereinrichtung 3 sodann die Modalität 2 entsprechend den Einstellungen E.

Entsprechend der Darstellung in FIG 6 sind weitere Ausgestaltungen möglich. So ist es insbesondere möglich, dass die Steuereinrichtung 3 den tatsächlichen Beginn der Ausführung des Untersuchungsauftrags U - also den Zeitpunkt T0' des Beginns der zu realisierenden Implementierung I' - in einem Schritt S41 an die Recheneinrichtung 1 übermittelt. In diesem Fall nimmt die Recheneinrichtung 1 den tatsächlichen Implementierungsbeginn T0' des Untersuchungsauftrags U durch die Modalität 2 in einem Schritt S51 entgegen. Zusätzlich kann ein Schritt S42 vorhanden sein, in dem die Steuereinrichtung 3 das tatsächliche Ende T1' der Ausführung des Untersuchungsauftrags U - also den Zeitpunkt des Endes der zu realisierenden Implementierung I' - an die Recheneinrichtung 1 übermittelt. In diesem Fall nimmt die Recheneinrichtung 1 das tatsächlichen Implementierungsende T1' durch die Modalität 2 in einem Schritt S52 entgegen. In einem Schritt S53 kann die Recheneinrichtung 1 daraufhin die für die Ausführung der Implementierung I' durch die Modalität 2 erforderliche Zeitdauer abschätzen. In einem Schritt S54 kann die Recheneinrichtung 1 sodann anhand der Abschätzung der Zeitdauer die betriebswirtschaftliche Bewertung B2 nachführen.

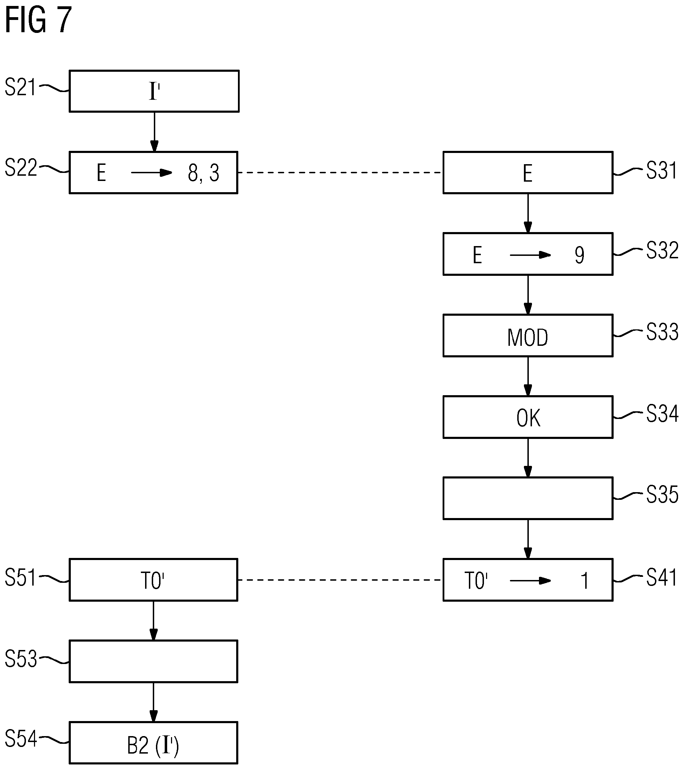

Alternativ ist es entsprechend der Darstellung in FIG 7 möglich, dass die Schritte S42 und S52 entfallen. In diesem Fall kann die Recheneinrichtung 1 im Schritt S53 ebenfalls eine für die Ausführung der einer Implementierung I' durch die Modalität 2 erforderliche Zeitdauer abschätzen. Im Unterschied zur Vorgehensweise von FIG 6 ist diese Zeitdauer jedoch nicht auf die Implementierung I' bezogen, für welche der Implementierungsbeginn T0' gültig ist, sondern auf die dieser Implementierung I' unmittelbar vorhergehende Implementierung I'.

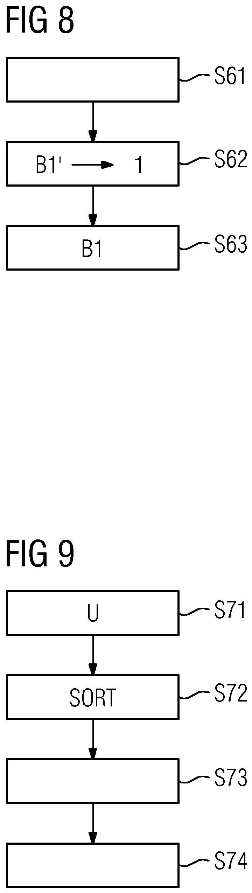

Neben der betriebswirtschaftlichen Bewertung B2 kann auch die medizinisch-technische Bewertung B1 nachgeführt werden. In diesem Fall kann die Recheneinrichtung 1 beispielsweise entsprechend der Darstellung in FIG 8 in einem Schritt S61 die mittels der Modalität 2 erfassten Daten - insbesondere Bilder - auswerten. Alternativ oder zusätzlich kann die Recheneinrichtung 1 in einem Schritt S62 von einem dritten Bediener 10 (der beispielsweise mit dem ersten Bediener 6 identisch sein kann) eine Bewertung B1' entgegennehmen. In einem Schritt S63 erfolgt sodann anhand der Auswertung des Schrittes S61 und/ oder der Bewertung B1' das Nachführen der medizinisch-technischen Bewertung B1. Die Vorgehensweise von FIG 8 kann beispielsweise nach Bedarf mit der Vorgehensweise von FIG 6 oder FIG 7 kombiniert werden, insbesondere sich an die dort erläuterten Vorgehensweisen anschließen.

Zum erstmaligen Ermitteln der betriebswirtschaftlichen Bewertungen B2 für die einzelnen möglichen, in der Datenbasis 7 gespeicherten Implementierungen I kann beispielsweise entsprechend der Darstellung in FIG 9 die Recheneinrichtung 1 in einem Schritt S71 auf Datensätze bereits ausgeführter Untersuchungsaufträge U zugreifen. Die bereits ausgeführten Untersuchungsaufträge U umfassen in aller Regel jeweils die untersuchte Person typisierende Daten typ, die Art der Untersuchung, die Modalität 2 und die Einstellungen E der Modalität 2 sowie die Anfangszeiten T0' des jeweiligen Untersuchungsauftrags U. Die bereits ausgeführten Untersuchungsaufträge U sind jedoch in der Regel zwar vorhanden, aber nicht miteinander verkettet. Die Recheneinrichtung 1 vergleicht daher insbesondere in einem Schritt S72 die Anfangszeiten T0' miteinander und ermittelt auf diese Weise - das heißt anhand der Anfangszeiten T0' - die Reihenfolge der bereits ausgeführten Untersuchungsaufträge U. Sodann ermittelt die Recheneinrichtung 1 in einem Schritt S73 anhand der Differenz unmittelbar aufeinanderfolgender Anfangszeiten T0' eine jeweilige geschätzte Implementierungsdauer für den jeweiligen bereits ausgeführten Untersuchungsauftrag U. Schließlich ermittelt die Recheneinrichtung 1 in einem Schritt S74 die jeweilige betriebswirtschaftliche Bewertung B2. Sie berücksichtigt hierbei die im Schritt S73 ermittelten Implementierungsdauern.

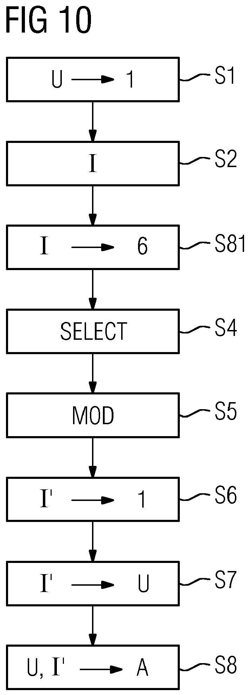

In vielen Fällen enthält die Datenbasis aufgeschlüsselt nach die zu untersuchende Person typisierenden Daten typ, Art der Untersuchung und Modalität 2 weitere Daten. Insbesondere kann sie für die weiteren einer jeweiligen möglichen Implementierung die zugeordneten Daten - also für die Einstellungen E der Modalität 2, die technisch-medizinische Bewertung B1 der jeweiligen Untersuchung und/oder die betriebswirtschaftliche Bewertung B2 der jeweiligen Untersuchung - jeweils einen üblichen Bereich enthalten. In diesem Fall ist entsprechend der Darstellung in FIG 10 der Schritt S3 von FIG 2 durch einen Schritt S81 ersetzt. Der Schritt S81 korrespondiert im Kern mit dem Schritt S3. Jedoch werden im Schritt S81 dem ersten Bediener 6 ein nicht nur die ermittelten möglichen Implementierungen I des Untersuchungsauftrags U angezeigt, sondern zusätzlich auch die Lage der Einstellungen E der Modalität 2 und/oder der technisch-medizinischen Bewertung B1 und/oder der betriebswirtschaftlichen Bewertung B2 innerhalb des jeweils üblichen Bereichs.

Zusammengefasst betrifft die vorliegende Erfindung somit folgenden Sachverhalt:
Eine Recheneinrichtung 1 nimmt von einem ersten Bediener 6 einen von einer bildgebenden medizintechnischen Einrichtung 2 (Modalität 2) auszuführenden Untersuchungsauftrag U entgegen. Der Untersuchungsauftrag U umfasst eine zu untersuchende Person individualisierende Daten ident, die Person typisierende Daten typ und die Art der Untersuchung festlegende Daten art. Die Recheneinrichtung 1 nimmt vom ersten Bediener 6 eine zu realisierende Implementierung I' des Untersuchungsauftrags U entgegen und ordnet diese Implementierung I' dem Untersuchungsauftrag U zu. Die Recheneinrichtung 1 ermittelt anhand der die Person typisierenden Daten typ und der die Art der Untersuchung festlegenden Daten art unter Zugriff auf eine Datenbasis 7 mögliche Implementierungen I des Untersuchungsauftrags U. Die Datenbasis 7 enthält aufgeschlüsselt nach die zu untersuchende Person typisierenden Daten typ, Art der Untersuchung, Modalität 2 und Einstellungen E der Modalität 2 jeweils eine technisch-medizinische Bewertung B1 der jeweiligen Untersuchung und eine betriebswirtschaftliche Bewertung B2 der jeweiligen Untersuchung. Die Recheneinrichtung 1 gibt die ermittelten möglichen Implementierungen I dem Bediener 6 zusammen mit den beiden Bewertungen B1, B2 zur Auswahl vor. Sie aktualisiert anhand der vom Bediener 6 entgegengenommenen zu realisierenden Implementierung I' einen Ablaufplan A für die Modalität 2, so dass der Ablaufplan A zusätzlich zu bereits im Ablaufplan A enthaltenen Implementierungen I' von anderen Untersuchungsaufträgen U auch den nunmehrigen Untersuchungsauftrag U und die zugeordnete zu realisierende Implementierung I' enthält.

Die vorliegende Erfindung weist viele Vorteile auf. Insbesondere kann auf einfache und zuverlässige Weise der Betrieb der Modalitäten 2 sowohl in betriebswirtschaftlicher als auch in technisch-medizinischer Hinsicht optimiert werden. Weiterhin kann der Vergleich mit den im Krankenhaus üblichen Vorgehensweisen werden jedes einzelnen Arbeitsschrittes sowohl vor als auch nach der Durchführung des Untersuchungsauftrags U vorgenommen werden. Die Beurteilung der zu realisierenden Implementierung I' kann lange vor der Durchführung erfolgen, insbesondere auch außerhalb der jeweiligen Modalität 2. Die Optimierung der Einstellungen E kann in beiden Dimensionen (technisch-medizinisch und betriebswirtschaftlich) erfolgen. Die erwarteten Implementierungsbeginne T0 können vorab ermittelt werden und bei der Auswahl der zu realisierenden Implementierung I' berücksichtigt werden. Nach der Durchführung des Untersuchungsauftrags U kann ein Feedback zur Optimierung erfolgen.

Obwohl die Erfindung im Detail durch das bevorzugte Implementierungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Festlegungsverfahren für eine von einer bildgebenden medizintechnischen Einrichtung (2) zu realisierende Implementierung (I') eines Untersuchungsauftrags (U),
- wobei eine Recheneinrichtung (1) von einem ersten Bediener (6) den von der bildgebenden medizintechnischen Einrichtung (2) auszuführenden Untersuchungsauftrag (U) entgegennimmt,
- wobei der Untersuchungsauftrag (U) eine zu untersuchende Person individualisierende Daten (ident), die zu untersuchende Person typisierende Daten (typ) und die Art der Untersuchung festlegende Daten (art) umfasst,
- wobei die Recheneinrichtung (1) vom ersten Bediener (6) eine zu realisierende Implementierung (I') des Untersuchungsauftrags (U) entgegennimmt und die zu realisierende Implementierung (I') des Untersuchungsauftrags (U) dem Untersuchungsauftrag (U) zuordnet,
- wobei die Recheneinrichtung (1) anhand der die zu untersuchende Person typisierenden Daten (typ) und der die Art der Untersuchung festlegenden Daten (art) des Untersuchungsauftrags (U) unter Zugriff auf eine Datenbasis (7) mögliche Implementierungen (I) des Untersuchungsauftrags (U) ermittelt,
- wobei die Datenbasis (7) aufgeschlüsselt nach die zu untersuchende Person typisierenden Daten (typ), Art der Untersuchung, bildgebender medizintechnischer Einrichtung (2) und Einstellungen (E) der bildgebenden medizintechnischen Einrichtung (2) jeweils eine technisch-medizinische Bewertung (B1) der jeweiligen Untersuchung und eine betriebswirtschaftliche Bewertung (B2) der jeweiligen Untersuchung enthält,
- wobei die Recheneinrichtung (1) die ermittelten möglichen Implementierungen (I) des Untersuchungsauftrags (U) dem ersten Bediener (6) zusammen mit der jeweiligen technisch-medizinischen Bewertung (B1) und der betriebswirtschaftlichen Bewertung (B2) zur Auswahl vorgibt und
- wobei die Recheneinrichtung (1) anhand der vom ersten Bediener (6) entgegengenommenen zu realisierenden Implementierung (I') des Untersuchungsauftrags (U) einen Ablaufplan (A) für die bildgebende medizintechnische Einrichtung (2) aktualisiert, so dass der Ablaufplan (A) zusätzlich zu bereits im Ablaufplan (A) enthaltenen Implementierungen (I') von anderen Untersuchungsaufträgen (U) auch den nunmehrigen Untersuchungsauftrag (U) und die zugeordnete zu realisierende Implementierung (I') des Untersuchungsauftrags (U) enthält.

2. Festlegungsverfahren nach Anspruch 1,
**dadurch gekennzeichnet,**
**dass** die Recheneinrichtung (1) anhand des Ablaufplans (A) für die bildgebende medizintechnische Einrichtung (2) die von dieser bildgebenden medizintechnischen Einrichtung (2) jeweils als nächstes zu realisierende Implementierung (I') ermittelt und die Einstellungen (E) dieser bildgebenden medizintechnischen Einrichtung (2) für die jeweils als nächstes zu realisierende Implementierung (I') einem zweiten Bediener (8) vorgibt.

3. Festlegungsverfahren nach Anspruch 2,
**dadurch gekennzeichnet,**
**dass** die Recheneinrichtung (1) mit einer Steuereinrichtung (3) für die bildgebende medizintechnische Einrichtung (2) datentechnisch verbunden ist und dass die Recheneinrichtung (1) die Einstellungen (E) der bildgebenden medizintechnischen Einrichtung (2) für die von dieser bildgebenden medizintechnischen Einrichtung (2) jeweils als nächstes zu realisierende Implementierung (I') aufgrund einer Bestätigung durch den zweiten Bediener (8) der Steuereinrichtung (3) vorgibt.

4. Festlegungsverfahren nach Anspruch 1, 2 oder 3,
**dadurch gekennzeichnet,**
**dass** die Recheneinrichtung (1) die ermittelten möglichen Implementierungen (I) des Untersuchungsauftrags (U) dem ersten Bediener (6) aufgeschlüsselt nach der jeweiligen bildgebenden medizintechnischen Einrichtung (2) vorgibt und dass die Recheneinrichtung (1) im Rahmen des Untersuchungsauftrags (U) vom ersten Bediener (6) auch eine Vorgabe entgegennimmt, mit welcher bildgebenden medizintechnischen Einrichtung (2) der Untersuchungsauftrag (U) ausgeführt werden soll.

5. Festlegungsverfahren nach einem der obigen Ansprüche,
**dadurch gekennzeichnet,**
**dass** die Recheneinrichtung (1) anhand des Ablaufplans (A) für die bildgebende medizintechnische Einrichtung (2), wie er der Recheneinrichtung (1) vor der Entgegennahme des Untersuchungsauftrags (U) vorliegt, für die möglichen Implementierungen (I) des Untersuchungsauftrags (U) jeweils einen möglichen Implementierungsbeginn (T0) ermittelt, zu dem die jeweilige mögliche Implementierung (I) des Untersuchungsauftrags (U) gestartet werden kann, und dass die Recheneinrichtung (1) dem ersten Bediener (6) zusammen mit den ermittelten möglichen Implementierungen (I) des Untersuchungsauftrags (U) auch den möglichen Implementierungsbeginn (T0) mit anzeigt.

6. Festlegungsverfahren nach einem der obigen Ansprüche,
**dadurch gekennzeichnet,**
**dass** die Recheneinrichtung (1) zur Entgegennahme der zu realisierenden Implementierung (I') vom ersten Bediener (6) eine Auswahl einer der möglichen Implementierungen (I) mit oder ohne Modifikation der jeweiligen möglichen Implementierung (I) entgegennimmt.

7. Festlegungsverfahren nach einem der obigen Ansprüche,
**dadurch gekennzeichnet,**
**dass** die Recheneinrichtung (1) den tatsächlichen Implementierungsbeginn (T0') des Untersuchungsauftrags (U) durch die bildgebende medizintechnische Einrichtung (2) und zusätzlich das tatsächliche Implementierungsende (T1') des Untersuchungsauftrags (U) durch die bildgebende medizintechnische Einrichtung (2) oder den tatsächlichen Implementierungsbeginn (T0') des nächsten Untersuchungsauftrags (U) durch die bildgebende medizintechnische Einrichtung (2) entgegennimmt und dass die Recheneinrichtung (1) aus diesen Daten die für die Ausführung der Implementierung (I') des Untersuchungsauftrags (U) durch die bildgebende medizintechnische Einrichtung (2) erforderliche Zeitdauer abschätzt und anhand der Abschätzung der Zeitdauer die betriebswirtschaftliche Bewertung (B2) nachführt.

8. Festlegungsverfahren nach einem der obigen Ansprüche,
**dadurch gekennzeichnet,**
**dass** die Recheneinrichtung (1) die mittels der bildgebenden medizintechnischen Einrichtung (2) erfassten Daten auswertet und/oder von einem dritten Bediener (10) eine Bewertung (B1') entgegennimmt und dass die Recheneinrichtung (1) die technisch-medizinische Bewertung (B1) anhand der Auswertung und/ oder der vom dritten Bediener (10) entgegengenommenen Bewertung (B1') nachführt.

9. Festlegungsverfahren nach einem der obigen Ansprüche,
**dadurch gekennzeichnet,**
- **dass** die Recheneinrichtung (1) auf Datensätze bereits ausgeführter Untersuchungsaufträge (U) zugreift,
- **dass** die bereits ausgeführten Untersuchungsaufträge (U) jeweils die untersuchte Person typisierende Daten (typ), die Art der Untersuchung, die bildgebende medizintechnische Einrichtung (2) und die Einstellungen (E) der bildgebenden medizintechnischen Einrichtung (2) sowie die Anfangszeiten (T0') der jeweiligen Untersuchungsaufträge (U) umfassen,
- **dass** die Recheneinrichtung (1) anhand der Anfangszeiten (T0') die Reihenfolge der bereits ausgeführten Untersuchungsaufträge (U) und für den jeweiligen bereits ausgeführten Untersuchungsauftrag (U) eine geschätzte Implementierungsdauer ermittelt und
- **dass** die Recheneinrichtung (1) die geschätzten Implementierungsdauern bei der Ermittlung der betriebswirtschaftlichen Bewertung (B2) berücksichtigt.

10. Festlegungsverfahren nach einem der obigen Ansprüche,
**dadurch gekennzeichnet,**
- **dass** die Datenbasis (7) aufgeschlüsselt nach die zu untersuchende Person typisierenden Daten (typ), Art der Untersuchung und bildgebender medizintechnischer Einrichtung (2) für die Einstellungen (E) der bildgebenden medizintechnischen Einrichtung (2) und/oder die technisch-medizinische Bewertung (B1) der jeweiligen Untersuchung und/oder die betriebswirtschaftliche Bewertung (B2) der jeweiligen Untersuchung jeweils einen üblichen Bereich enthält und
- **dass** die Recheneinrichtung (1) dem ersten Bediener (6) zusammen mit den ermittelten möglichen Implementierungen (I) des Untersuchungsauftrags (U) auch die Lage der Einstellungen (E) der bildgebenden medizintechnischen Einrichtung (2) und/oder der technisch-medizinischen Bewertung (B1) der jeweiligen Untersuchung und/oder der betriebswirtschaftlichen Bewertung (B2) der jeweiligen Untersuchung innerhalb des jeweils üblichen Bereichs mit anzeigt.

11. Computerprogramm, das Maschinencode (5) umfasst, der von einer Recheneinrichtung (1) abarbeitbar ist, wobei die Abarbeitung des Maschinencodes (5) durch die Recheneinrichtung (1) bewirkt, dass die Recheneinrichtung (1) ein Festlegungsverfahren nach einem der obigen Ansprüche ausführt.

12. Recheneinrichtung, wobei die Recheneinrichtung mit einem Computerprogramm (4) nach Anspruch 11 programmiert ist, so dass sie im Betrieb ein Festlegungsverfahren nach einem der Ansprüche 1 bis 10 ausführt.

## Geänderte Patentansprüche

### Geänderte Patentansprüche gemäss Regel 137(2) EPÜ.

1. Festlegungsverfahren für eine von einer bildgebenden medizintechnischen Einrichtung (2) zu realisierende Implementierung (I') eines Untersuchungsauftrags (U),
- wobei eine Recheneinrichtung (1) von einem ersten Bediener (6) den von der bildgebenden medizintechnischen Einrichtung (2) auszuführenden Untersuchungsauftrag (U) entgegennimmt,
- wobei der Untersuchungsauftrag (U) eine zu untersuchende Person individualisierende Daten (ident), die zu untersuchende Person typisierende Daten (typ) und die Art der Untersuchung festlegende Daten (art) umfasst,
- wobei die Recheneinrichtung (1) vom ersten Bediener (6) eine zu realisierende Implementierung (I') des Untersuchungsauftrags (U) entgegennimmt und die zu realisierende Implementierung (I') des Untersuchungsauftrags (U) dem Untersuchungsauftrag (U) zuordnet,
- wobei die Recheneinrichtung (1) anhand der die zu untersuchende Person typisierenden Daten (typ) und der die Art der Untersuchung festlegenden Daten (art) des Untersuchungsauftrags (U) unter Zugriff auf eine Datenbasis (7) mögliche Implementierungen (I) des Untersuchungsauftrags (U) ermittelt,
- wobei die möglichen Implementierungen (I) jeweils die Modalität (2), mit welcher der Untersuchungsauftrag (U) ausgeführt werden soll, und die Einstellungen (E) der entsprechenden Modalität (2) umfassen,
- wobei die Datenbasis (7) aufgeschlüsselt nach die zu untersuchende Person typisierenden Daten (typ), Art der Untersuchung, bildgebender medizintechnischer Einrichtung (2) und Einstellungen (E) der bildgebenden medizintechnischen Einrichtung (2) jeweils eine technisch-medizinische Bewertung (B1) der jeweiligen Untersuchung und eine betriebswirtschaftliche Bewertung (B2) der jeweiligen Untersuchung enthält,
- wobei die Recheneinrichtung (1) die ermittelten möglichen Implementierungen (I) des Untersuchungsauftrags (U) dem ersten Bediener (6) zusammen mit der jeweiligen technisch-medizinischen Bewertung (B1) und der betriebswirtschaftlichen Bewertung (B2) zur Auswahl vorgibt und
- wobei die Recheneinrichtung (1) anhand der vom ersten Bediener (6) entgegengenommenen zu realisierenden Implementierung (I') des Untersuchungsauftrags (U) einen Ablaufplan (A) für die bildgebende medizintechnische Einrichtung (2) aktualisiert, so dass der Ablaufplan (A) zusätzlich zu bereits im Ablaufplan (A) enthaltenen Implementierungen (I') von anderen Untersuchungsaufträgen (U) auch den nunmehrigen Untersuchungsauftrag (U) und die zugeordnete zu realisierende Implementierung (I') des Untersuchungsauftrags (U) enthält.

2. Festlegungsverfahren nach Anspruch 1,
**dadurch gekennzeichnet,**
**dass** die Recheneinrichtung (1) anhand des Ablaufplans (A) für die bildgebende medizintechnische Einrichtung (2) die von dieser bildgebenden medizintechnischen Einrichtung (2) jeweils als nächstes zu realisierende Implementierung (I') ermittelt und die Einstellungen (E) dieser bildgebenden medizintechnischen Einrichtung (2) für die jeweils als nächstes zu realisierende Implementierung (I') einem zweiten Bediener (8) vorgibt.

3. Festlegungsverfahren nach Anspruch 2,
**dadurch gekennzeichnet,**
**dass** die Recheneinrichtung (1) mit einer Steuereinrichtung (3) für die bildgebende medizintechnische Einrichtung (2) datentechnisch verbunden ist und dass die Recheneinrichtung (1) die Einstellungen (E) der bildgebenden medizintechnischen Einrichtung (2) für die von dieser bildgebenden medizintechnischen Einrichtung (2) jeweils als nächstes zu realisierende Implementierung (I') aufgrund einer Bestätigung durch den zweiten Bediener (8) der Steuereinrichtung (3) vorgibt.

4. Festlegungsverfahren nach Anspruch 1, 2 oder 3,
**dadurch gekennzeichnet,**
**dass** die Recheneinrichtung (1) die ermittelten möglichen Implementierungen (I) des Untersuchungsauftrags (U) dem ersten Bediener (6) aufgeschlüsselt nach der jeweiligen bildgebenden medizintechnischen Einrichtung (2) vorgibt und dass die Recheneinrichtung (1) im Rahmen des Untersuchungsauftrags (U) vom ersten Bediener (6) auch eine Vorgabe entgegennimmt, mit welcher bildgebenden medizintechnischen Einrichtung (2) der Untersuchungsauftrag (U) ausgeführt werden soll.

5. Festlegungsverfahren nach einem der obigen Ansprüche,
**dadurch gekennzeichnet,**
**dass** die Recheneinrichtung (1) anhand des Ablaufplans (A) für die bildgebende medizintechnische Einrichtung (2), wie er der Recheneinrichtung (1) vor der Entgegennahme des Untersuchungsauftrags (U) vorliegt, für die möglichen Implementierungen (I) des Untersuchungsauftrags (U) jeweils einen möglichen Implementierungsbeginn (TO) ermittelt, zu dem die jeweilige mögliche Implementierung (I) des Untersuchungsauftrags (U) gestartet werden kann, und dass die Recheneinrichtung (1) dem ersten Bediener (6) zusammen mit den ermittelten möglichen Implementierungen (I) des Untersuchungsauftrags (U) auch den möglichen Implementierungsbeginn (TO) mit anzeigt.

6. Festlegungsverfahren nach einem der obigen Ansprüche,
**dadurch gekennzeichnet,**
**dass** die Recheneinrichtung (1) zur Entgegennahme der zu realisierenden Implementierung (I') vom ersten Bediener (6) eine Auswahl einer der möglichen Implementierungen (I) mit oder ohne Modifikation der jeweiligen möglichen Implementierung (I) entgegennimmt.

7. Festlegungsverfahren nach einem der obigen Ansprüche,
**dadurch gekennzeichnet,**
**dass** die Recheneinrichtung (1) den tatsächlichen Implementierungsbeginn (TO') des Untersuchungsauftrags (U) durch die bildgebende medizintechnische Einrichtung (2) und zusätzlich das tatsächliche Implementierungsende (T1') des Untersuchungsauftrags (U) durch die bildgebende medizintechnische Einrichtung (2) oder den tatsächlichen Implementierungsbeginn (T0') des nächsten Untersuchungsauftrags (U) durch die bildgebende medizintechnische Einrichtung (2) entgegennimmt und dass die Recheneinrichtung (1) aus diesen Daten die für die Ausführung der Implementierung (I') des Untersuchungsauftrags (U) durch die bildgebende medizintechnische Einrichtung (2) erforderliche Zeitdauer abschätzt und anhand der Abschätzung der Zeitdauer die betriebswirtschaftliche Bewertung (B2) nachführt.

8. Festlegungsverfahren nach einem der obigen Ansprüche,
**dadurch gekennzeichnet,**
**dass** die Recheneinrichtung (1) die mittels der bildgebenden medizintechnischen Einrichtung (2) erfassten Daten auswertet und/oder von einem dritten Bediener (10) eine Bewertung (B1') entgegennimmt und dass die Recheneinrichtung (1) die technisch-medizinische Bewertung (B1) anhand der Auswertung und/ oder der vom dritten Bediener (10) entgegengenommenen Bewertung (B1') nachführt.

9. Festlegungsverfahren nach einem der obigen Ansprüche,
**dadurch gekennzeichnet,**
- **dass** die Recheneinrichtung (1) auf Datensätze bereits ausgeführter Untersuchungsaufträge (U) zugreift,
- **dass** die bereits ausgeführten Untersuchungsaufträge (U) jeweils die untersuchte Person typisierende Daten (typ), die Art der Untersuchung, die bildgebende medizintechnische Einrichtung (2) und die Einstellungen (E) der bildgebenden medizintechnischen Einrichtung (2) sowie die Anfangszeiten (T0') der jeweiligen Untersuchungsaufträge (U) umfassen,
- **dass** die Recheneinrichtung (1) anhand der Anfangszeiten (T0') die Reihenfolge der bereits ausgeführten Untersuchungsaufträge (U) und für den jeweiligen bereits ausgeführten Untersuchungsauftrag (U) eine geschätzte Implementierungsdauer ermittelt und
- **dass** die Recheneinrichtung (1) die geschätzten Implementierungsdauern bei der Ermittlung der betriebswirtschaftlichen Bewertung (B2) berücksichtigt.

10. Festlegungsverfahren nach einem der obigen Ansprüche,
**dadurch gekennzeichnet,**
- **dass** die Datenbasis (7) aufgeschlüsselt nach die zu untersuchende Person typisierenden Daten (typ), Art der Untersuchung und bildgebender medizintechnischer Einrichtung (2) für die Einstellungen (E) der bildgebenden medizintechnischen Einrichtung (2) und/oder die technisch-medizinische Bewertung (B1) der jeweiligen Untersuchung und/oder die betriebswirtschaftliche Bewertung (B2) der jeweiligen Untersuchung jeweils einen üblichen Bereich enthält und
- **dass** die Recheneinrichtung (1) dem ersten Bediener (6) zusammen mit den ermittelten möglichen Implementierungen (I) des Untersuchungsauftrags (U) auch die Lage der Einstellungen (E) der bildgebenden medizintechnischen Einrichtung (2) und/oder der technisch-medizinischen Bewertung (B1) der jeweiligen Untersuchung und/oder der betriebswirtschaftlichen Bewertung (B2) der jeweiligen Untersuchung innerhalb des jeweils üblichen Bereichs mit anzeigt.

11. Computerprogramm, das Maschinencode (5) umfasst, der von einer Recheneinrichtung (1) abarbeitbar ist, wobei die Abarbeitung des Maschinencodes (5) durch die Recheneinrichtung (1) bewirkt, dass die Recheneinrichtung (1) ein Festlegungsverfahren nach einem der obigen Ansprüche ausführt.

12. Recheneinrichtung, wobei die Recheneinrichtung mit einem Computerprogramm (4) nach Anspruch 11 programmiert ist, so dass sie im Betrieb ein Festlegungsverfahren nach einem der Ansprüche 1 bis 10 ausführt.
